# NEUE EUROPÄISCHE PATENTSCHRIFT

(11) **EP 0 340 511 B2**
(45) Veröffentlichungstag und Bekanntmachung des Hinweises auf die Entscheidung über den Einspruch: **29.01.1997**
(45) Hinweis auf die Patenterteilung: 27.01.1993
(21) Anmeldenummer: 89106745.6
(22) Anmeldetag: 14.04.1989
(51) Int. Cl.: G01N 33/52, C12Q 1/34, C12Q 1/37, C12Q 1/48

(54) **Trägergebundenes mehrkomponentiges Nachweissystem zur kolorimetrischen Bestimmung esterolytisch oder/und proteolytisch aktiver Inhaltsstoffe von Körperflüssigkeiten**
Carrier-bound multicomponent detection system for the colorimetric determination of esterolytic or/and proteolytic substances of body fluids
Système à plusieurs composants attaché à un support pour l'analyse colorimétrique de substances d'estérolyse et/ou de protéolyse actives dans des fluides corporels

(30) Priorität: 22.04.1988 DE 3813503
(43) Veröffentlichungstag der Anmeldung: 08.11.1989
(73) Patentinhaber: BOEHRINGER MANNHEIM GMBH, 68298 Mannheim (DE)
(72) Erfinder: Backhaus, Jürgen, Dr., D-6803 Edingen (DE); Mangold, Dieter, Dr., D-6701 Maxdorf (DE); Knappe, Wolfgang-Reinhold, Dr., D-6842 Bürstadt 1 (DE)

(56) Entgegenhaltungen:
- EP-A- 39 880
- EP-A- 64 710
- EP-A- 137 521
- EP-A- 157 361
- EP-A- 157 362
- EP-A- 158 204
- EP-A- 200 540
- EP-A- 244 825
- FR-A- 2 514 511
- JP-A-59 030 063
- JP-A-62 275 698

## Beschreibung

Die vorliegende Erfindung betrifft ein trägergebundenes, mehrkomponentiges Nachweissystem zur kolorimetrischen Bestimmung esterolytisch oder/und proteolytisch aktiver Inhaltsstoffe von Körperflüssigkeiten.

Dem Nachweis esterolytisch oder/und proteolytisch aktiver Inhaltsstoffe von Körperflüsigkeiten kommt eine große Bedeutung zu. Insbesondere für die Diagnostik von Erkrankungen der Niere und des Urogenitaltraktes ist es wichtig, Leukozyten anhand der ihnen innewohnenden esterolytischen oder/und proteolytischen Aktivität in Urin nachzuweisen, Hierfür haben sich trägergebundene Nachweissysteme, z.B. in Form von Test, streifen als ganz besonders vorteilhaft erwiesen, weil diese nur kurz in die zu untersuchende flüssige Probe getaucht zu werden brauchen und dann anschließend nach dem Herausziehen des Streifens aus der Probe innerhalb kurzer Zeit eine Ermittlung der Zahl der Leukozyten pro µl Probe z.B. aufgrund einer Verfärbung erlauben. So ist eine saubere, einfache und schnelle Möglichkeit zum Nachweis solcher Inhaltsstoffe in Körperflüssigkeiten gegeben, ohne daß hierzu komplizierte, zeit- und kostenaufwendige Manipulationen mit oder an der Probe durchgeführt werden müssen.

Als vorteilhafte Nachweissysteme zur kolorimetrischen Bestimmung esterolytisch oder/und proteolytisch aktiver Inhaltsstoffe von Körperflüssigkeiten, wie z.B. von Leukozyten in Urin sind aus dem Stand der Technik vor allem sogenannte Mehrkomponentensysteme bekannt. Das sind solche Reagenzien-Systeme, die aus mehreren chemischen Substanzen bestehen, von denen mindestens eine durch esterolytisch oder/und proteolytisch aktive Inhaltsstoffe von Körperflüssigkeiten chemisch so verändert wird, daß das Reaktionsprodukt direkt mit einer oder in einer Reaktionsfolge mit mehreren der übrigen Komponenten des Nachweis-Systems zu einer farbigen Substanz reagiert, die dann kolorimetrisch bestimmt werden kann. Die Menge der gebildeten farbigen Substanz ist ein Maß für die Menge an esterolytisch oder/und proteolytisch aktiven Inhaltsstoffen in der untersuchten Probe.

Besonders geeignet für diese Art des Nachweises esterolytisch oder/und proteolytisch aktiver Inhaltsstoffe in Körperflüssigkeiten sind z.B. Zweikomponenten-Systeme, bei denen zuerst ein Ester durch die nachzuweisenden Inhaltsstoffe gespalten und die entstandene Hydroxyverbindung dann durch eine weitere reaktive Substanz zu einem Farbstoff umgesetzt wird. Trägergebundene Mehrkomponentensysteme dieses Prinzips sind z.B. aus der europäischen Patentanmeldung 0 039 880 bekannt.

Diese Patentanmeldung betrifft u.a. trägergebundene Nachweissysteme für die Bestimmung esterolytisch oder/und proteolytisch aktiver Inhaltsstoffe in Körperflüssigkeiten, wie z.B. Leukozyten in Urin, bei denen ein durch diesen Inhaltsstoff spaltbarer Ester und ein Diazoniumsalz als Reaktionspartner für die durch Esterspaltung erzeugte Hydroxyverbindung gemeinsam auf denselben Reagenzträger aufgebracht sind. Das gemeinsame Vorliegen sämtlicher Komponenten des Nachweissystems auf ein und demselben Reagenzträgerschien insofern optimal, als es offensichtlich so für gewährleistet gehalten wurde, daß das durch Reaktion mit den esterolytisch oder/und proteolytisch aktiven Inhaltsstoffen entstandene Produkt rasch zu einem farbigen Endprodukt weiterreagieren kann.

Ein rasches und vollständiges Ablaufen der Nachweisreaktion bildet die Grundlage für einen empfindlichen Test. Im Falle der vorgenannten Nachweissysteme liegt die Nachweisgrenze für Leukozyten in Urin bei Ablesung des Wertes nach ca. 2 Minuten bei etwa 15-25 Leukozyten/µl Urin. Aus Sicht des Arztes ist es jedoch wünschenswert, Nachweismittel zur Verfügung zu haben, die noch empfindlicher sind. Längere Wartezeiten bis zur Ablesung des zu bestimmenden Wertes sind unerwünscht.

Aus der EP-A-0 064 710 ist ein Testmittel bekannt, welches eine die flüssige Probe verzögert aufsaugende Schicht und eine Schicht, die die für die Nachweisreaktion erforderlichen Reagenzien enthält, aufweist.

Aus EP-A-0244825 ist bekannt, daß bei Cholesterintests auf Redoxbasis die Trennung einer Farbvorstufe und eines Elektronenüberträgers zu einer erhöhten Sensitivität und Genauigkeit führt.

In FR-A-2514511 ist die Erhöhung der Empfindlichkeit von lmmuntests durch Ausführung auf einem Teststreifen beschrieben.

Aus EP-A-0200540 ist ein analytisches Element zur Bestimmung von Kreatinkinase bekannt, bei dem Kreatinphosphat und eine mit H₂O₂ reagierende Indikatorsubstanz in getrennten Schichten vorliegen.

Auch in EP-A0137521 wird ein solches vielschichtiges analytisches Element beschrieben, bei dem noch zusätztich eine Oxidase-haltige Schicht vorhanden ist.

Der Erfindung lag daher die Aufgabe zugrunde, für die medizinische Diagnose ein trägergebundenes mehrkomponentiges Nachweissystem zur kolorimetrischen Bestimmung esterolytisch oder/und proteolytisch aktiver Inhaltsstoffe von Körperflüssigkeiten zur Verfügung zu stellen, welches die Empfindlichkeit bekannter trägergebundener Nachweissysteme übertrifft, ohne mit dem Nachteil einer längeren Wartezeit bis zur Ablesung des vom Nachweissystem angezeigten Wertes verbunden zu sein.

Demgemäß wurde das eingangs definierte trägergebundene mehrkomponentige Nachweissystem zur kolorimetrischen Bestimmung esterolytisch oder/und proteolytisch aktiver Inhaltsstoffe von Körperflüssigkeiten enthaltend eine durch den Inhaltsstoff esterolytisch oder/und proteolytisch spaltbare Komponente und eine mit dem Produkt dieser Reaktion zu einer farbigen Substanz reagierende Komponente, gefunden, das dadurch gekennzeichnet ist, daß die Komponenten des Nachweissystems in verschiedenen, einen Flüssigkeitsaustausch zwischen sich ermöglichenden Reagenzträgern vorliegen und sich der Reagenzträger mit der spaltbaren Komponente zwischen einem Kunststoffträger und dem Reagenzträger mit der mit dem Produkt der Reaktion zu einer farbigen Substanz reagierenden Komponente befindet.

Gegenstand der Erfindung ist außerdem ein Verfahren zum Nachweis von Leukozyten in Urin enthaltend
- in Kontakt-Bringen von Urin mit mindestens einer esterolytisch oder / und proteolytisch spaltbaren Komponente und mindestens einem Diazoniumsalz und
- Reaktion der durch die Leukozyten gespaltenen Komponente mit dem Diazoliumsalz,
dadurch gekennzeichnet, daß das Diazoliumsalz von einem ersten Reagenzträger und die spaltbare Komponente von einem zweiten Reagenzträger mindestens teilweise gelöst und die Komponenten miteinander vermischt werden,
sowie die Verwendung eines vorstehend beschriebenen Reagenzträgers zum Nachweis esterolytisch oder / und proteolytisch aktiver Inhaltsstoffe von Körperflüssigkeiten.

Als mehrkomponentige Nachweissysteme zur kolorimetrischen Bestimmung esterolytisch oder/und proteolytisch aktiver Inhaltsstoffe von Körperflüssigkeiten kommen hierbei die aus dem Stand der Technik hierfür bekannten Reagenzsysteme in Frage. Als besonders vorteilhaft haben sich solche mehrkomponentigen Nachweissystem erwiesen, die einen esterolytisch oder/und proteolytisch spaltbaren Ester und ein Diazoniumsalz enthalten. Solche Systeme haben ihre Eignung vor allem zur Bestimmung von Leukozyten in Urin bewiesen. Sie können aber auch zum Nachweis proteolytischer Enzyme wie z.B. Elastase, Chymotrypsin oder Trypsin in wässrigen Lösungen bzw. in Körperflüssigkeiten, wie z. B. Vollblut, Serum, Liquor, Pankreassekret oder wässrigen Stuhlextrakten dienen.

Ganz besonders vorteilhaft sind die in der europäischen Patentanmeldung 0 039 880 genannten Reagenziensysteme erfindungsgemäß einsetzbar. Hierzu werden mindestens ein esterolytisch oder/und proteolytisch spaltbarer Ester und mindestens ein Diazoniumsalz der dort beschriebenen Art auf getrennten Reagenzträgern aufgebracht.

Neben den unmittelbar chemisch in die Nachweisreaktion eingreifenden Komponenten des Nachweissystems wie, z.B. Ester und Diazoniumsalz kann dieses auch noch weitere Hilfsstoffe wie, z.B. Puffersubstanzen, Netzmittel, Stabilisatoren oder/und Aktivatoren enthalten. Solche Hilfsstoffe sind z. B. ebenfalls aus der europäischen Patentanmeldung 0 039 880 bekannt. Sie bilden jedoch keine Komponenten des Nachweissystems im Sinne der vorliegenden Erfindung.

Als Reagenzträger können alle üblicherweise hierfür einsetzbaren Materialien verwendet werden, wie z.B. saugfähige oder quellbare, poröse oder nicht poröse Materialien oder auch solche, die sich bei Kontakt mit der zu untersuchenden Flüssigkeit darin auflösen. Beispielhaft seien genannt Cellulose, Filterpapier, Kunstfaser- oder Glasfaservlies, Polyvinylester-, Polyamidfilme oder auch Filme aus z. B. Xanthan-Gum.

Erfindungsgemäße trägergebundene Nachweismittel enthalten die Komponenten des Nachweissystems in verschiedenen Reagenzträgern. Die in die Nachweisreaktion direkt chemisch eingreifenden Reagenzien werden erst nach der Kontaktierung mit der zu untersuchenden Flüssigkeit durch mindestens teilweises in Lösung gehen darin miteinander vermischt. Die verschiedenen Reagenzbräger sind dabei zwar getrennt, dennoch aber so angeordnet, daß zwischen ihnen ein Flüssigkeitsaustausch möglich ist. In einer bevorzugten Ausführungsform des erfindungsgemäßen trägergebundenen Nachweismittels sind z. B. die, die jeweiligen Komponenten des Nachweissystems wie z. B.Ester und Diazoniumsalz enthaltenden Reagenzträger übereinanderliegend, quasi als Stapel angeordnet. Zur besseren Handhabung dieses Reagenzträgerstapels kann dieser z. B. auf einem Kunststoffträger, wie eine steife Folie angebracht sein. Das Fixieren des Stapels auf dem Kunststoffträger kann beispielsweise durch Einnetzen, durch Seitenverklebung oder durch Aufkleben eines Reagenzträgers auf den Kunststoffträger und punktuelles Aufkleben des weiteren Reagenzträgers auf den vorangehenden erfolgen. Zum besseren Aufsaugen der zu untersuchenden Flüssigkeit, kann ein solcher Reagenzträgerstapel beispielsweise auch durch ein Leervlies unterlegt sein.

Als Materialien für einen direkt auf einem Kunststoffträger liegenden Reagenzträger sind Kunststofffilme wie Polyester- oder Polyamidfilme oder saugfähige Materialien, wie z. B. ein Cellulosevlies besonders gutgeeignet. Für darüber angeordnete Reagenzträger sind beispielsweise Gewebe, dünne durchscheinende, poröse Materialien, durchscheinende Filme oder mit der zu untersuchenden Flüssigkeit sich auflösende Materialien besonders vorteilhaft. Durch Kontakt mit der zu untersuchenden Flüssigkeit, beispielsweise durch Eintauchen solcher trägergebundenen Nachweismittel oder durch dosierte Aufgabe der zu untersuchenden Flüssigkeit, werden die in den Reagenzträgern vorliegenden Komponenten des Nachweissystems mindestens teilweise in Lösung gebracht, insgesamt durch den Lösungsvorgang vermischt und letztendlich die Nachweisreaktion bei Anwesenheit esterolytisch oder/und proteolytisch aktiver Inhaltsstoffe in Gang gesetzt.

Überraschenderweise hat es sich gezeigt, daß das erfindungsgemäße trägergebundene Nachweismittel gegenüber bisher bekannten Mitteln bis zu mehr als 100 % empfindlicher ist. Bei zwei Minuten Wartezeit können 5 Leukozyten pro µl Probe kolorimetrisch bestimmt werden. Werte ab 10 Leukozyten pro µl Probe sind sogar schon 1 Minute nach Benetzung des trägergebundenen Nachweismittels ablesbar.

Die Erfindung wird durch das nachfolgende Beispiel näher erläutert. Sie soll jedoch nicht als darauf eingeschränkt gelten.

### Beispiel 1

### Empfindlichkeit des Tests zum Nachweis von Leukozyten in Urin

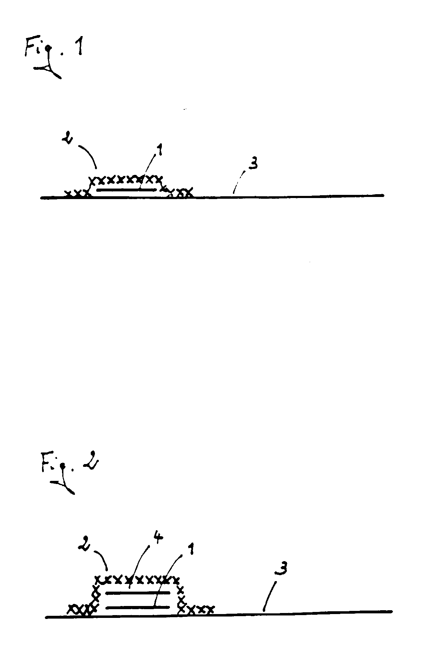

A) Trägergebundenes Nachweissystem des Standes der Technik gemäß Figur 1 mit Einmalimprägnierung bezüglich Ester und Diazoniumsalz:
Ein Papiervlies 23 SL der Firma Schleicher & Schüll, Dassel, Bundesrepublik Deutschland, wurde in der folgenden Weise mit den Hilfsstoffen und Komponenten des Nachweissystems imprägniert:
a) Vortränkung mit einer Tränklösung, die in 1 l Wasser 65 g Borsäure und 21 g Natriumhydroxid enthielt und mit 1 N Salzsäure auf pH 8.0 eingestellt worden war. Anschließend Trocknung des getränkten Vlieses.
b) Haupttränkung des nach a) hergestellten puffergetränkten Vlieses mit einer Lösung die in 1 l Ethanol 0,35 g 2-Methoxy-4-(N-Morpholino)-benzol-diazonium-tetrachlorozinkat, 0.78 g 3-(N-Toluol-4'-sulfonyl-L-alanyloxy)-indol, 81,3 g Phosphorsäuretrimorpholid und 21,7 ml Decanol enthielt und anschließende Trocknung.
Das so imprägnierte Vlies wurde in Quadrate von 6 x6 mm geschnitten und die so hergestellten Reagenzträger (1) jeweils mittels eines Nylonnetzes (2) NY75HC (Fadendicke 60 um) der Firma Züricher Beuteituchfabrik AG, Zürich, Schweiz, auf einem 12.6 cm langen, 6 mm breiten und 1 mm dicken Polystyrolstreifen (3) befestigt. Das Nylonnetz wurde hierzu in der Hitze bei etwa 175° C mit dem Kunststoffstreifen teilweise verschmolzen.
B) Trägergebundenes Nachweissystem gemäß Figur 1 mit getrennter Imprägnierung Dezüglich Ester und Diazoniumsalz:
Ein Papiervlies 23 SL der Firma Schleicher & Schüll, Dassel, Bundesrepublik Deutschland, wurde in der folgenden Weise mit den Hilfsstoffen und Komponenten des Nachweissystems imprägniert:
a) Vortränkung mit einer Tränklösung, die in 1 l Wasser 65 g Borsäure und 21 g Natriumhydroxid enthielt und mit 1 N Salzsäure auf pH 8.0 eingestellt worden war. Anschließend Trocknung des getränkten Vlieses.
b) Tränkung des nach a) hergestellten puffergetränkten Vlieses mit einer Lösung, die in 1 l Ethanol 0.78 g 3-(N-Toluol-4'-sulfonyl-L-alanyloxy)-indol, 81.3 g Phosphorsäuretrimorpholid und 21.7 ml Decanol enthielt und anschließend Trocknung.
c) Tränkung des nach a) und b) imprägnierten Vlieses mit einer Lösung, die in 1 l Wasser 0.37 g 2-Methoxy-4-(N-morpholino)-benzol-diazonium-tetrachlorzinkat und 0.22 g Weinsäure enthielt und anschließende Trocknung.
Mit dem so imprägnierten Papiervlies wurde analog A) ein trägergebundenes Nachweissystem gemäß Figur 1 hergestellt.
C) Erfindungsgemäßes Nachweissystem (Fig. 2):
l) Ein Papiervlies 23 SL der Firma Schleicher & Schüll, Dassel, Bundesrepublik Deutschland, wurde in der folgenden Weise imprägniert:
   a) Vortränkung mit einer Tränklösung, die in 1 l Wasser 65 g Borsäure und 21 g Natriumhydroxid enthielt und mit 1 N Salzsäure auf pH 8,0 eingestellt worden war. Anschließend Trocknung des getränkten Vlieses.
   b) Haupttränkung des nach a) hergestellten puffergetränkten Vlieses mit einer Lösung die in 1 l Ethanol 0,78 g 3-(N-Toluol-4'-sulfonyl-L-alanyloxy)-indol, 81,3 g Phosphorsäuretrimorpholid und 21.7 ml Decanol enthielt und anschließend Trocknung.
II) Ein Nylongewebe NY 20 HC (Fadendicke 20 µm) der Firma Züricher Beuteltuchfabrik AG, Zürich, Schweiz, wurde mit einer Lösung, die in 1 l Wasser 3,78 g 2-Methoxy-4-(N-morpholino)-benzol-diazoniumtetrachlorozinkat und 2.25 g Weinsäure enthielt, getränkt und anschließend getrocknet.
Das imprägnierte Papiervlies aus 1) wurde in Quadrate von 6 x 6 mm geschnitten und ergab den Reagenzträger (1). Das in II hergestellte Nylongewebe wurde in ebenso große Quadrate geschnitten und ergab Reagenzträger (4). Die Reagenzträger 1) und (4) wurden als Stapel mittels eines Nylonnetzes (2) NY75HC (Fadendicke 60 µm) der Firma Züricher Beuteltuchfabrik, Zürich, Schweiz, auf einem 12.6 cm langen, 6 mm breiten und 1 mm dicken Polystyrolstreifen (3) fixiert. Das Nylonnetz selbst wurde auf dem Kunststoffstreifen durch teilweises Verschmelzen in der Hitze bei etwa 175 °C befestigt.
D) Leukozytenhaltige Lösungen wurden durch Aufstockung leukozytenfreien Harns mit aus Blut isolierten Leukozyten hergestellt. Folgende Konzentrationen lagen zum Testen der Nachweissysteme vor: 0,5, 10, 20 und 40 Leukozyten pro µl Harn.
Die in A), B) und C) hergestellten Teststreifen wurden in die leukozytenfreien und leukozytenhaltigen Harnproben getaucht und nach 2 Minuten bezüglich Farbbildung visuell abgelesen oder remissionsphotometrisch vermessen. Mit den in A), B) und C) beschriebenen Nachweissystemen wurden folgende Ergebnisse erhalten:

| | Farbbildung bei Nachweissystem | | | Zunahme der Remission, gemessen mit Urotron RL-9 in Remissionseinheiten (Boehringer Mannheim GmbH) | | |
|---|---|---|---|---|---|---|
| Zahl der Leukozyten/ µl Probe | A | B | C | A | B | C |
| 0 | - | - | - | 0 | 0 | 0 |
| 5 | - | - | + | 0 | 0 | 4 |
| 10 | - | - | + | 0 | 0 | 9 |
| 20 | + | + | + | 6 | 5 | 17 |
| 40 | + | + | + | 14 | 13 | 31 |

Während bei den Nachweissystemen A und B (Stand der Technik) 2 Minuten nach Probenkontakt die Nachweisgrenze für Leukozyten bei ca. 20 Leukoyzten/µl Probe lag, wurden mit dem erfindungsgemäßen Nachweissystem C schon 5 Leukozyten/µl Probe angezeigt.

Bereits nach 1 Minute kann mit Nachweissystem C eine Konzentration von 10 Leukozyten/µl Probe visuell oder mittels Remissionsmessung nachgewiesen werden.
Mit Nachweissystemen A und B liegt bei dieser kurzen Zeit die Nachweisgrenze bei mehr als 20 Leukozyten/µl.

## Patentansprüche

1. Trägergebundenes mehrkomponentiges Nachweissystem zur colorimetrischen Bestimmung esterolytisch oder/und proteolytisch aktiver Inhaltsstoffe von Körperflüssigkeiten enthaltend eine durch den Inhaltsstoff esterolytisch oder/und proteolytisch spaltbare Komponente und eine mit dem Produkt dieser Reaktion zu einer farbigen Substanz reagierende Komponente, wobei die Komponenten in verschiedenen einen Flüssigkeitsaustausch zwischen sich ermöglichenden Reagenzträgern vorliegen und wobei sich der Reagenzträger mit der spaltbaren Komponente zwischen einem Kunststoffträger und dem Reagenzträger mit der mit dem Produkt der Reaktion zu einer farbigen Substanz reagierenden Komponente befindet.

2. Nachweissystem gemäß Anspruch 1, dadurch gekennzeichnet, daß als Komponenten mindestens ein esterolytisch oder/und proteolytisch spaltbarer Ester und mindestens ein Diazoniumsalz auf getrennten Reagenzträgern aufgebracht sind.

3. Verfahren zum Nachweis von Leukozyten in Urin enthaltend
- in-Kontakt-Bringen von Urin mit mindestens einer esterolytisch oder/und proteolytisch spaltbaren Komponente und mindestens einem Diazoniumsalz und
- Reaktion der durch die Leukozyten gespaltenen Komponente mit dem Diazoniumsalz,
dadurch gekennzeichnet, daß das Diazoniumsalz von einem ersten Reagenzträger und die spaltbare Komponente von einem zweiten Reagenzträger mindestens teilweise gelöst und die Komponenten miteinander vermischt werden.

4. Verfahren gemäß Anspruch 3, dadurch gekennzeichnet, daß das Verfahren mittels eines trägergebundenen mehrkomponentigem Nachweissystems durchgeführt wird, bei dem der zweite Reagenzträger zwischen einem Kunststoffträger und dem ersten Reagenzträger angeordnet ist.

5. Verwendung eines Reagenzträgers nach Anspruch 1 zum Nachweis von esterolytisch oder/und proteolytisch aktiven Inhaltsstoffen von Körperflüssigkeiten.

## Claims

1. Carrier-bound multicomponent detection system for the colorimetric determination of esterolytically- and/or proteolytically-active component materials of body fluids containing a component cleavable esterolytically and/or proteolytically by the component material and a component reacting with the product of this reaction to give a coloured substance , whereby the components are present in different reaction carriers making possible a liquid exchange between them and whereby the reagent carrier with the cleavable component is present between a synthetic material carrier and the reagent carrier with the component reacting with the product of the reaction to give a coloured substance.

2. Detection system according to claim 1, characterised in that, as components, at least one esterolytically- and/or proteolytically-cleavable ester and at least one diazonium salt are applied to separate reagent carriers.

3. Process for the detection of leukocytes in urine comprising
- bringing into contact of urine with at least one esterolytically- and/or proteolytically-cleavable component and at least one diazonium salt and
- reaction of the component cleaved by the leukocytes with the diazonium salt,
characterised in that the diazonium salt is dissolved at least partly from the first reagent carrier and the cleavable component from the second reagent carrier and the components are mixed with one another.

4. Process according to claim 3, characterised in that the process is carried out by means of a carrier-bound multicomponent detection system in which the second reagent carrier is arranged between a synthetic material carrier and the first reagent carrier.

5. Use of a reagent carrier according to claim 1 for the detection of esterolytically- and/or proteolytically-active component materials of body fluids.

## Revendications

1. Système de détection à plusieurs composants, lié à un support, destiné à la détermination colorimétrique de substances ayant une activité estérolytique et/ou protéolytique contenues dans des fluides corporels, comportant un composant pouvant être coupé par estérolyse et/ou protéolyse et un composant réagissant avec le produit de cette réaction en le transformant en une substance colorée, où les composants sont disposés sur différents supports de réactifs permettant un échange de liquide entre eux et dans lequel le support de réactif portant les composants pouvant être coupés se trouve entre un support en matière plastique et le support de réactif portant le composant réagissant avec le produit de la réaction en le transformant en une substance colorée.

2. Système de détection selon la revendication 1, caractérisé en ce que comme composants on applique, sur des supports de réactif séparés, au moins un ester pouvant être coupé par estérolyse et/ou protéolyse et au moins un sel de diazonium.

3. Procédé de détection de leucocytes dans l'urine, consistant:
- à mettre en contact l'urine avec au moins un composant pouvant être coupé par estérolyse et/ou protéolyse et au moins un sel de diazonium, et
- à faire réagir le composant coupé par les leucocytes avec le sel de diazonium,
caractérisé en ce que le sel de diazonium est dissous au moins partiellement à partir d'un premier support de réactif et le composant pouvant être coupé est dissous au moins partiellement à partir d'un second support de réactif et que les composants sont mélangés entre eux.

4. Procédé selon la revendication 3, caractérisé en ce que le procédé est effectué au moyen d'un système de détection à plusieurs composants dans lequel le second support de réactif est disposé entre un support en matière plastique et le premier support de réactif.

5. Utilisation d'un support de réactif selon la revendication 1 pour la détection de substances ayant une activité estérolytique et/ou protéolytique, contenues dans des fluides corporels.
